# EUROPEAN PATENT APPLICATION

(11) **EP 4 331 665 A1**
(43) Date of publication of application: **06.03.2024**
(21) Application number: 22795404.7
(22) Date of filing: 25.03.2022
(51) Int. Cl.: A61P 3/04, C07D 233/84, A61K 9/48, A61K 36/07, A61K 31/4164, A23L 33/175, A23L 33/20

(54) **COMPOSITION FOR PROMOTING LIPOLYSIS**

(30) Priority: 26.04.2021 JP 2021074319
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); MURAYAMA, Norihito, Soraku-gun, Kyoto 619-0284 (JP)
(74) Representative: Vossius & Partner Patentanwälte Rechtsanwälte mbB
(86) International application number: PCT/JP2022/014207
(87) International publication number: WO 2022/230486

(57) **Abstract**

The present invention aims to provide a composition for promoting lipolysis and a method of promoting lipolysis. The present invention relates to a composition for promoting lipolysis, the composition containing L-ergothioneine or a salt thereof as an active ingredient.

## Description

### TECHNICAL FIELD

The present invention relates to a composition for promoting lipolysis. The present invention also relates to, for example, a method of promoting lipolysis.

### BACKGROUND ART

Factors such as high-calorie diet and lack of exercise cause systemic or regional accumulation of excessive fat, resulting in obesity. Obesity is a cause of various lifestyle-related diseases and is also a social problem. Thus, studies have been conducted on components effective in preventing or reducing obesity and applicable to foods for specified health uses, foods with function claims, pharmaceutical products, and the like.

Ergothioneine is an amino acid found in mushrooms and the like. L-ergothioneine is present in nature. According to Patent Literature 1, the peritesticular fat weight and the triglyceride (neutral fat) level in plasma were lower in mice orally administered with ergothioneine than in mice not orally administered with ergothioneine.

### CITATION LIST

### - Patent Literature

Patent Literature 1: JP 2011-102286 A

### SUMMARY OF INVENTION

### - Technical Problem

Examples of approaches for preventing or reducing obesity include a method of inhibiting fat absorption, a method of promoting lipolysis, and a method of promoting fat burning. For example, the term "lipolysis" refers to a reaction process in which neutral fat (triglyceride) is hydrolyzed into glycerol and free fatty acid. The reaction proceeds due to the action of lipase in the adipose tissue. The term "fat burning" refers to a process in which fatty acid produced by lipolysis or the like is converted into energy. Thus, promoting breakdown of accumulated fats (lipolysis) and promoting fat burning are different mechanisms. Patent Literature 1 is silent about an action of L-ergothioneine to promote lipolysis.

The present invention aims to provide a composition for promoting lipolysis. The present invention also aims to provide a method of promoting lipolysis.

### - Solution to Problem

As a result of extensive studies to solve the above problem, the present inventors found that L-ergothioneine has an action to promote lipolysis.

Specifically, the present invention relates to, but is not limited to, a composition for promoting lipolysis, a method of promoting lipolysis, and the like.
(1) A composition for promoting lipolysis, containing L-ergothioneine or a salt thereof as an active ingredient.
(2) The composition according to (1) above, wherein the composition is an oral composition.
(3) The composition according to (1) or (2) above, wherein the composition is a food or beverage.
(4) The composition according to any one of (1) to (3) above, wherein an amount of L-ergothioneine or a salt thereof in the composition per adult daily intake is 2 to 50 mg in terms of L-ergothioneine.
(5) A method of promoting lipolysis, including administering L-ergothioneine or a salt thereof.
(6) Use of L-ergothioneine or a salt thereof for promoting lipolysis.

### - Advantageous Effects of Invention

The present invention can provide a composition for promoting lipolysis. The present invention can also provide a method of promoting lipolysis.

### BRIEF DESCRIPTION OF DRAWINGS

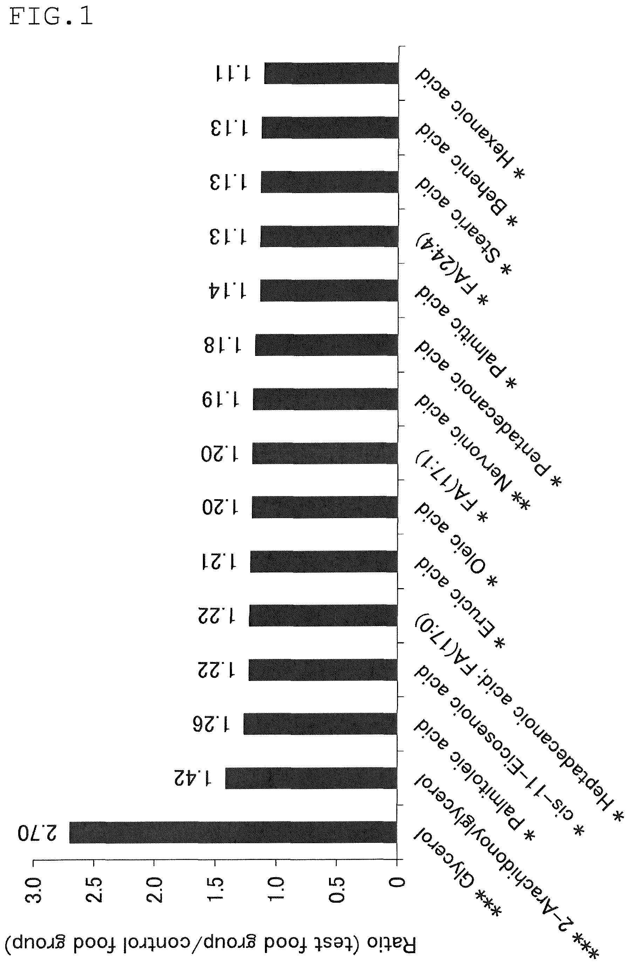

FIG. 1 is a graph showing blood metabolome measurements of a control food group and a test food group at the time of testing on week 4 in the form of ratios (test food group/control food group).

### DESCRIPTION OF EMBODIMENTS

The composition for promoting lipolysis of the present invention contains L-ergothioneine or a salt thereof as an active ingredient. The composition for promoting lipolysis of the present invention is sometimes also simply referred to as "the composition of the present invention".

L-ergothioneine is one of the amino acids.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*), shiitake (binomial name: *Lentinula edodes*), hen-of-the-wood (binomial name: *Grifola Frondosa*), reishi mushuroom (binomial name: *Ganoderma lucidum*), lion's mane mushroom (binomial name: *Hericium erinaceus*), Yanagimatsutake (binomial name: *Agrocybe aegerita*), girolle (binomial name: *Cantharellus cibarius*)*,* porcini (binomial name: *Boletus edulis*), and common morel (binomial name: *Morchella esculenta*). When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

As described later in Examples, the blood glycerol concentration was significantly increased when L-ergothioneine was ingested compared to when no L-ergothioneine was ingested. The blood free fatty acid (e.g., palmitoleic acid) concentration was also increased when L-ergothioneine was ingested compared to when no L-ergothioneine was ingested.

Hydrolysis of neutral fat (triglyceride) in fat cells results in glycerol and free fatty acids which are then released into the blood. For example, blood glycerol is used as a marker to detect lipolysis, and an increase in blood glycerol concentration indicates that lipolysis is promoted. An increase in blood free fatty acid concentration also indicates that lipolysis is promoted. Thus, L-ergothioneine or a salt thereof has an action to promote lipolysis and can be used as an active ingredient to promote lipolysis.

An increase in blood glycerol concentration can also be interpreted as that extracellular release of glycerol produced by breakdown of neutral fat in tissue such as adipose tissue is promoted. Thus, L-ergothioneine or a salt thereof has an action to promote extracellular release of glycerol.

L-ergothioneine or a salt thereof has an action to increase the blood glycerol concentration. Glycerol produced by breakdown of neutral fat is released into the blood and circulates in the body. Glycerol is a substance that acts as an osmotically active substance (osmolyte) for intracellular osmoregulation in organisms such as mammals. According to a report (Goulet et al., Journal of Sports Science and Medicine (2020)1, 96-102), glycerol ingestion increases the water content in the body. Thus, an effect of maintaining or increasing the water content in the body is likely to be achieved by increasing the blood glycerol concentration.

In one embodiment, the composition for promoting lipolysis of the present invention can be preferably used as a composition for promoting lipolysis in humans. The composition of the present invention is useful in preventing or ameliorating a condition or disease which can be effectively prevented or ameliorated by promoting lipolysis. In one embodiment, the composition of the present invention can be used, for example, for preventing or reducing obesity by promoting lipolysis.

Preventing a condition or disease encompasses preventing the onset of a condition or disease, delaying the onset of a condition or disease, reducing the incidence of a condition or disease, reducing the onset risk of a condition or disease, and the like. Ameliorating a condition or disease encompasses helping a subject recover from a condition or disease, alleviating a symptom of a condition or disease, favorably changing a symptom of a condition or disease, delaying or preventing the progress of a condition or disease, and the like.

The composition of the present invention is applicable for therapeutic use (medical use) and non-therapeutic use (non-medical use). The "non-therapeutic" is a concept that does not include medical activities, i.e., a concept that does not include methods of surgery, therapy, or diagnosis of humans.

The composition for promoting lipolysis of the present invention can be provided as an agent or the like, for example, but it is not limited thereto. The agent can be directly provided as a composition or can be provided as a composition containing the agent. In one embodiment, the composition for promoting lipolysis of the present invention can also be referred to as an agent for promoting lipolysis.

In order to sufficiently obtain the effect of the present invention, preferably, the composition of the present invention is an oral composition. The oral composition may be a food or beverage, an oral pharmaceutical product, an oral quasi-pharmaceutical product, or feed, preferably a food or beverage or an oral pharmaceutical product, still more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in foods, beverages, pharmaceutical products, quasi-pharmaceutical products, feed, and the like can be used. When the composition of the present invention is provided as a food or beverage, a pharmaceutical product, a quasi-pharmaceutical product, feed, or the like, the production method is not limited, and any common method can be used for production.

For example, when the composition of the present invention is provided as a food or beverage, various types of food or beverages can be provided by adding a component usable in food or beverages (e.g., a food material or an optional food additive) to L-ergothioneine or a salt thereof. Non-limiting examples of the food or beverages include general foods and beverages, health foods, health beverages, foods with function claims, foods for specified health uses, dietary supplements, and foods and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion. The pharmaceutical product and the quasi-pharmaceutical product may be for non-human animals.

When the composition of the present invention is provided as feed, L-ergothioneine or a salt thereof is simply added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of L-ergothioneine or a salt thereof in the composition of the present invention is not limited and can be set according to the composition form and the like.

The amount of L-ergothioneine or a salt thereof, for example, in terms of L-ergothioneine, in the composition of the present invention is preferably 0.0001 wt% or more, more preferably 0.001 wt% or more and is preferably 90 wt% or less, more preferably 50 wt% or less. In one embodiment, the amount of L-ergothioneine or a salt thereof in terms of L-ergothioneine in the composition is preferably 0.0001 to 90 wt%, more preferably 0.001 to 50 wt%. In one embodiment, when the composition of the present invention is provided as a food or beverage, a pharmaceutical product, a quasi-pharmaceutical product, feed, or the like, preferably, the amount of L-ergothioneine or a salt thereof is in the above ranges.

In the case of L-ergothioneine, the amount in terms of L-ergothioneine or an expression similar thereto refers to the amount of L-ergothioneine. In the case of a salt of L-ergothioneine, the amount in terms of L-ergothioneine or an expression similar thereto refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides a lipolysis-promoting effect, and may be appropriately set according to the administration form, administration method, body weight of a subject, and the like.

In one embodiment, when orally feeding or administering the composition of the present invention to a human (adult) as a subject, the intake of L-ergothioneine or a salt thereof in terms of L-ergothioneine per day is preferably 2 mg or more, more preferably 5 mg or more, still more preferably 10 mg or more, and is preferably 50 mg or less, more preferably 25 mg or less, still more preferably 20 mg or less. In one embodiment, when orally feeding or administering the composition of the present invention to a human (adult), the intake of L-ergothioneine or a salt thereof in terms of L-ergothioneine per day is preferably 2 to 50 mg, more preferably 5 to 25 mg, still more preferably 5 to 20 mg, particularly preferably 10 to 20 mg. Preferably, the above amount of the composition is fed or administered in one or more portions per day, for example, in one portion or several portions (for example, two or three portions) per day. In one embodiment of the present invention, the composition of the present invention may be an oral composition for feeding or administering the above amount of L-ergothioneine or a salt thereof per 60 kg body weight per day to a human.

In one embodiment, when the composition of the present invention is parenterally administered to a human (adult), the administration amount of L-ergothioneine or a salt thereof in terms of L-ergothioneine per day is, for example, preferably 2 to 50 mg, more preferably 5 to 25 mg, still more preferably 5 to 20 mg, particularly preferably 10 to 20 mg.

In one embodiment, in the case of a human (adult), preferably, the above amount of L-ergothioneine or a salt thereof per 60 kg body weight per day is fed or administered.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is likely to result in a higher lipolysis-promoting effect. Thus, in a preferred embodiment, the composition of the present invention is continuously fed or administered. In one embodiment of the present invention, the composition of the present invention is continuously fed or administered preferably for at least one week, more preferably for at least two weeks.

The composition of the present invention may be fed or administered to any subject (subject to administration). The subject is preferably a human or non-human mammal, more preferably a human.

The composition of the present invention may be fed or administered to a subject needing or wanting to promote lipolysis. The subject to administration may be, for example, a human needing or wanting to prevent or reduce obesity. In one embodiment, the subject to administration may be a subject with obesity. In one embodiment, the subject to administration of the composition of the present invention may be a healthy person. The composition of the present invention can be used on a healthy person, for example, for purposes such as preventing a condition or disease which can be effectively prevented or ameliorated by promoting lipolysis.

The composition for promoting lipolysis of the present invention may be labeled with a function claim based on promotion of lipolysis. For example, the composition of the present invention may be labeled with a function claim such as "promoting lipolysis".

In one embodiment of the present invention, preferably, the composition of the present invention is a food or beverage labeled with such a function claim. The label may be one indicating use of the composition for obtaining the function. The label may be directly attached to the composition or may be attached to a container or a package of the composition.

The present invention encompasses the following method and use:
a method of promoting lipolysis, including feeding or administering L-ergothioneine or a salt thereof; and
use of L-ergothioneine or a salt thereof for promoting lipolysis.

Feeding or administering L-ergothioneine or a salt thereof to a subject can promote lipolysis. Preferably, L-ergothioneine or a salt thereof is orally fed or administered.

The method may be therapeutic or non-therapeutic. The use may be therapeutic or non-therapeutic.

In the method and use, L-ergothioneine or a salt thereof, preferred embodiments thereof, and the like are as described above for the composition for promoting lipolysis of the present invention. In the method and use, preferably, L-ergothioneine or a salt thereof is fed or administered to a subject one or more times per day, for example, one to several times (e.g., two to three times) per day. The use is preferably for humans or non-human mammals, more preferably for humans. In one embodiment, L-ergothioneine or a salt thereof can be used to prevent or reduce obesity by promoting lipolysis.

In the method and use, L-ergothioneine or a salt thereof is simply used in an amount (effective amount) that provides the lipolysis-promoting effect. Preferred administration amounts of L-ergothioneine or a salt thereof, preferred subjects to administration, and the like, are as described above for the composition for promoting lipolysis of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can be used to produce a food or beverage, a pharmaceutical product, a quasi-pharmaceutical product, feed, or the like for use in promoting lipolysis. In one embodiment, the present invention also encompasses use of L-ergothioneine or a salt thereof for producing a composition for promoting lipolysis.

The present invention also encompasses L-ergothioneine or a salt thereof for use in promoting lipolysis.

All academic literature and patent literature cited herein are incorporated herein by reference.

### EXAMPLES

The present invention is described in further detail below with reference to examples, but the scope of the present invention is not limited thereto.

### <Example 1>

### (Blood metabolome evaluation study on human)

In order to evaluate the influence of supplements containing ergothioneine on the human blood metabolome, a placebo-controlled, randomized, double-blind, parallelgroup study was conducted on subjects including male and female adults (46 subjects in a test food group; 46 subjects in a control food group; 92 subjects in total). In this study, one capsule containing 20 mg ergothioneine (test food) or one capsule not containing ergothioneine (control food) was fed per day for four weeks. The blood metabolome of each subject was measured for pre-testing before starting the study (before starting ingestion of the test food or control food). After ingestion of the test food or control food for four weeks, the blood metabolome of each subject was measured again (testing on week 4). The blood metabolome was measured by a capillary electrophoresis time-of-flight mass spectrometry (CE-TOF-MS) available from Human Metabolome Technologies, Inc.

### (Food for evaluation)

Two types of food for evaluation, which were indistinguishable in appearance, flavor, and the like, were used.
· Test food: capsule containing a test substance (20 mg L-ergothioneine)
· Control food: capsule containing no test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

For each of lipid metabolism-related metabolites and lipids of various kinds for which significant changes were observed by the blood metabolome measurement, a ratio (test food group/control food group) was determined from the average of measurements (blood concentrations) of the test food group and the control food group at the time of testing on week 4. FIG. 1 is a graph showing blood metabolome measurements of the test food group and the control food group at the time of testing on week 4 in the form of ratios (test food group/control food group). The metabolomes shown in FIG. 1 are described below. These are substances produced by lipolysis.
Glycerol, 2-Arachidonoylglycerol, Palmitoleic acid, cis-11-Eicosenoic acid, Heptadecanoic acid; FA (17:0), Erucic acid, Oleic acid, Fatty acid (FA) (17:1), Nervonic acid, Pentadecanoic acid, Palmitic acid, Fatty acid (FA) (24:4), Stearic acid, Behenic acid, Hexanoic acid

A ratio (test food group/control food group) of higher than 1.0 shown in FIG. 1 indicates a significant increase in blood concentration in the test food group compared to the control food group. A 2-sample t-test (***: p < 0.001, **: p <0.01, *: p <0.05, vs. control food group) was performed for significance testing between the groups. Metabolomes for which significance was already observed between the test food group and the control food group at the time of pre-testing were excluded. According to the results, the concentration of glycerol as a marker for detecting lipolysis in the test food group was increased with an increment of 2.7 times the concentration of glycerol in the control food group. This indicates that ingestion of the test food promoted lipolysis. In fact, in the test food group, the blood concentration was increased in almost all other lipid metabolism-related metabolites and lipids of various kinds. Ingestion of the test food significantly promoted lipolysis as a whole.

L-ergothioneine was found to have an action to promote lipolysis.

## Claims

1. A composition for promoting lipolysis, comprising
L-ergothioneine or a salt thereof as an active ingredient.

2. The composition according to claim 1,
wherein the composition is an oral composition.

3. The composition according to claim 1 or 2,
wherein the composition is a food or beverage.

4. The composition according to any one of claims 1 to 3,
wherein an amount of L-ergothioneine or a salt thereof in the composition per adult daily intake is 2 to 50 mg in terms of L-ergothioneine.

5. A method of promoting lipolysis, comprising
administering L-ergothioneine or a salt thereof.

6. Use of L-ergothioneine or a salt thereof for promoting lipolysis.
